# EUROPEAN PATENT APPLICATION

(11) **EP 4 282 946 A1**
(43) Date of publication of application: **29.11.2023**
(21) Application number: 21920968.1
(22) Date of filing: 20.01.2021
(51) Int. Cl.: C12M 3/00

(54) **EMBRYO CULTURING DEVICE AND IMAGING DEVICE THEREFOR**

(71) Applicant: Asada Ladies Clinic, Nagoya-shi, Aichi, 450-0002 (JP)
(72) Inventor: FUKUNAGA Noritaka, Nagoya-shi, Aichi 450-0002 (JP)
(74) Representative: Kuhnen & Wacker Patent- und Rechtsanwaltsbüro PartG mbB
(86) International application number: PCT/JP2021/001845
(87) International publication number: WO 2022/157854

(57) **Abstract**

An embryo culture device that accommodates a plurality of trays that each accommodate a treatment egg having been subjected to insemination treatment, and keeps the plurality of trays in a culture environment includes: a culture unit that keeps the plurality of trays in the culture environment; and a plurality of imaging units that are each provided in association with each one of the trays held in the culture unit, and continuously or intermittently image locations of the trays. An image captured by each of the imaging units is stored in a storage unit per tray. The image stored per recess part is corrected, and the corrected image is displayed on a display unit.

## Description

### BACKGROUND

### Field

The present disclosure relates to a technique that cultures an embryo.

### Related Art

There is an increasing social demand for infertility care, and various devices that culture inseminated ova (hereinafter simply referred treatment eggs) under microscopes or the like are proposed or sold. In recent years, devices that each include a camera provided in such a culture device to image a treatment egg during cultured, and to display a captured image on an external display as needed are also becoming popular (see, for example, JP 2012-39929 A). In such an embryo culture device, a treatment egg is placed in a tray (also referred to as a dish) that stably holds the treatment egg, is transported into a visual field of the camera at a predetermined interval together with the tray, and is imaged. An embryologist can decide whether or not fertilization succeeds, by observing this image, and consequently does not need to take the treatment egg outside of the culture device only to observe.

On the other hand, in response to increase in fertility treatment, there is a demand that one embryo culture device cultures multiple treatment eggs, and a plurality of trays are accommodated in the embryo culture device. In such a case, the following problems occur when treatment eggs subjected to insemination treatment are imaged. These problems occur not only in the context where fertilized eggs are subjected to microscope insemination processing, but also when treatment eggs subjected to in-vitro insemination processing by some methods are cultured.
[1] A time interval for imaging one treatment egg becomes long. Assuming that transporting and imaging a tray that holds one treatment egg take, for example, two minutes, one embryo culture device that accommodates 10 trays can obtain only one image every 20 minutes per tray. As the number of trays to be accommodated becomes greater, a transportation path to an imaging device becomes longer, and more time is taken, and therefore an interval taken until next imaging is performed after imaging gradually increases. Therefore, the number of trays that can be accommodated in the one embryo culture device is limited.
[2] A structure that transports a tray becomes complicated, and readily goes out of order. If a failure occurs in a transportation mechanism and transportation stops, this tray is placed outside of the original culture space held in a constant temperature and constant humidity environment and treatment eggs in the tray are susceptible to a negative influence. Keeping the transportation path and an imaging portion together in the constant temperature and constant humidity environment can keep such negative influences at a minimum, yet causes a problem that a device configuration becomes larger accordingly.
[3] The one imaging device images the treatment eggs on the plurality of trays, and therefore management of captured images becomes complicated. Furthermore, in a case where a treatment egg is not imaged, if there is some delay until this case is noticed, the image of the treatment egg that is being cultured becomes insufficient, which makes it difficult to accurately make, for example, a fertilization determination.

### SUMMARY

The present disclosure has been made to solve at least part of the above-described problem, and can achieve the following aspects and application embodiments.
(1) Various aspects of the present disclosure will be described below. The first aspect is an aspect of an embryo culture device that keeps in a culture environment, a treatment egg having been subjected to insemination treatment. This embryo culture device includes: a culture unit that accommodates a plurality of trays each including a plurality of recess parts that can each accommodate a treatment egg, and keeps the plurality of trays in the culture environment; a plurality of imaging units that are each provided in association with each one of the trays held in the culture unit, and continuously or intermittently image at least two or more of the plurality of recess parts among locations of the trays at once; a storage unit that divides and stores an image captured by each of the imaging units per area including the plurality of recess parts; a correction unit that corrects the stored image per recess part; and a determination unit that determines using the corrected image whether or not the treatment egg is fertilized. This embryo culture device can image the plurality of recess parts that can accommodate the treatment eggs at once using the imaging unit provided per tray, then divide the image per recess part, and correct each divided image. Consequently, the image of the treatment egg accommodated in each recess part is corrected while imaging the plurality of recess parts at once, so that it is possible to more accurately perform fertilization determination.

(12) The second aspect is an aspect of an imaging device that is attached to a culture unit of an embryo culture device that accommodates a plurality of trays each including a plurality of recess parts that can each accommodate a treatment egg having been subjected to insemination treatment, and keeps the plurality of trays in the culture environment. This imaging device includes a plurality of imaging units that are provided in association with each one of the plurality of trays held in the culture unit, and continuously or intermittently image at least two or more of the plurality of recess parts among locations of the trays at once; a storage unit that divides and stores an image captured by each of the imaging units per area including the plurality of recess parts; a correction unit that corrects the stored image per recess part; and a display unit that displays the corrected image. This imaging device can image the plurality of recess parts that can accommodate the treatment eggs at once using the imaging unit provided per tray, then divide the image per recess part, and correct each divided image. Consequently, the image of the treatment egg accommodated in each recess part is corrected while imaging the plurality of recess parts at once, and is displayed, so that it is possible to accurately learn the situations of treatment eggs.

The present disclosure can be carried out not only as such an embryo culture device and an imaging device for the embryo culture device, but also as other aspects. The present disclosure can be carried out as, for example, an embryo culture method, a method for attaching the imaging device to the embryo culture device, a method for manufacturing the embryo culture device, a method for observing treatment eggs accommodated in the embryo culture device, or a fertilization determination device or a method thereof for the treatment eggs.

### BRIEF DESCRIPTION OF THE DRAWINGS

FIG. 1 is a plan view of an embryo culture device according to a first embodiment;
FIG. 2 is a perspective view illustrating part of the embryo culture device;
FIG. 3 is a plan view illustrating an embodiment of culture chamber;
FIG. 4 is an explanatory view illustrating an electric configuration of the embryo culture device that includes a time lapse unit;
FIG. 5 is a plan view illustrating an example of a tray that accommodates a treatment egg;
FIG. 6 is an explanatory view for describing a relationship between the time lapse unit and a well of the tray;
FIG. 7A is an explanatory view illustrating an internal configuration of the time lapse unit;
FIG. 7B is an explanatory view illustrating a configuration of a camera module;
FIG. 8 is a flowchart illustrating a culture unit control routine;
FIG. 9 is an explanatory view illustrating display examples at a normal time and an abnormal time of a display module;
FIG. 10 is a flowchart illustrating a time lapse control processing routine;
FIG. 11 is an explanatory view illustrating an example of imaging and correction of a treatment egg in the well;
FIG. 12 is a schematic view illustrating a principle of adjusting a focus position in a case where a camera unit captures an image;
FIG. 13 is an explanatory view of a dish including a microwell that accommodates a treatment egg;
FIG. 14 is a schematic view illustrating a configuration example in a case where the camera unit is provided on a side surface of the culture chamber; and
FIG. 15 is an explanatory view illustrating a relationship between the well and the treatment egg accommodated in the well when the tray is seen from the side surface.

### DESCRIPTION OF THE PREFERRED EMBODIMENTS

### A. First Embodiment

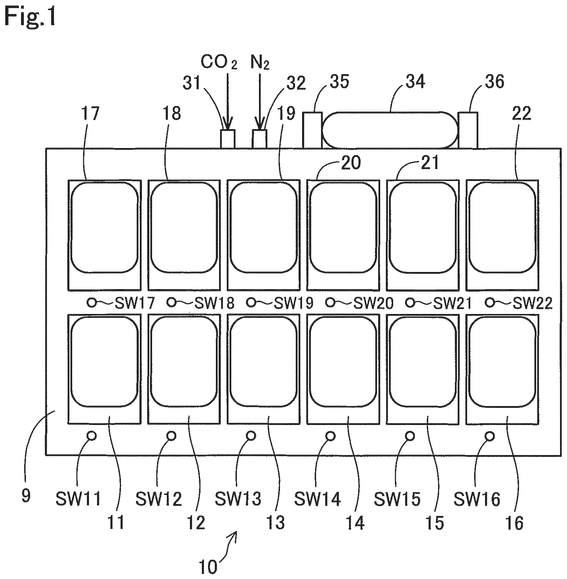

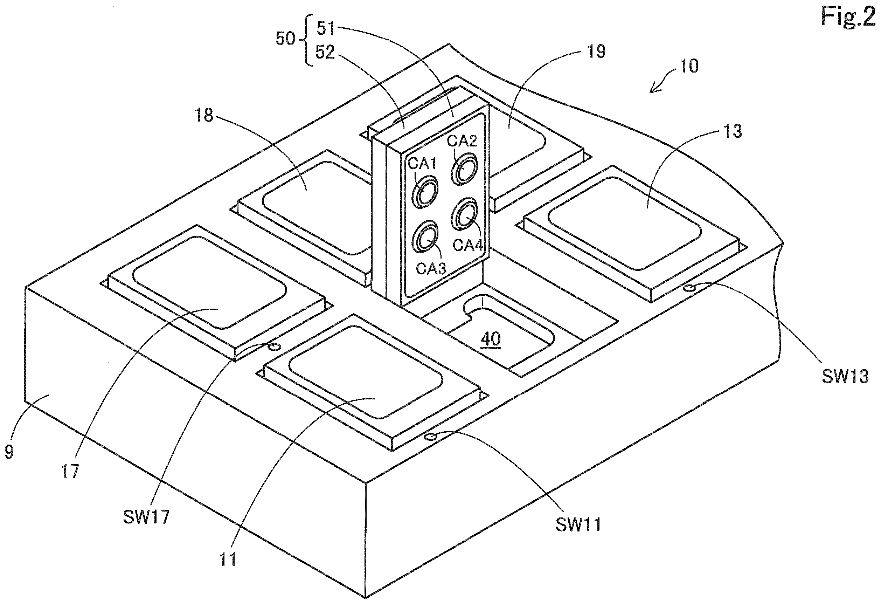

The first embodiment will be described. FIG. 1 is a plan view of an embryo culture device (also referred to as an incubator) 10, and FIG. 2 is a perspective view illustrating part of the embryo culture device 10. This embryo culture device 10 cultures an egg (hereinafter, referred to as a treatment egg) subjected to in-vitro fertilization treatment for a certain period in a predetermined culture environment, that is, at a constant temperature and a constant humidity. The treatment egg is not necessarily a fertilized egg, and therefore is cultured for a predetermined period by the embryo culture device 10. Note that there may be a case where in-vitro fertilization treatment is intracytoplasmic sperm injection performed under a microscope, or a case where in-vitro fertilization treatment is general ex-vivo fertilization performed by placing an egg and a sperm together in a predetermined container. A method for fertilizing a treatment egg to be cultured does not matter.

As illustrated in FIG. 1, this embryo culture device 10 includes 12 culture units 11 to 22. The number of culture units does not matter. A configuration and a function of each of the culture units 11 to 22 will be described later. Two front and rear rows of every six of these culture units 11 to 22 are provided in a width direction in a flat case main body 9. The respective culture units 11 to 22 are provided in association with switches SW11 to SW22, respectively. By operating these switches SW11 to SW22, a time lapse unit 50 of each of the corresponding culture units 11 to 22 is opened or closed by an unillustrated drive mechanism. The culture units 11 to 22 may also adopt a structure that is manually opened and closed.

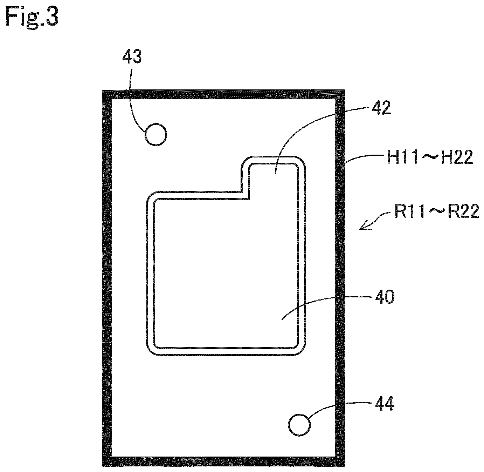

FIG. 2 illustrates that the time lapse unit 50 is in an opened state in the culture unit 12. The time lapse unit 50 includes a camera module 51 and a display module 52. The camera module 51 and the display module 52 have the same dimension, and are integrally formed. The camera module 51 is provided with four camera units CA1 to CA4. Structures and functions of these camera units CA1 to CA4 will be described later. The respective culture units 11 to 22 include culture chambers R11 to R22 (see FIG. 3) prepared therein, respectively, and the time lapse units 50 of the respective culture chambers R11 to R22 also function as lids for the corresponding culture chambers R11 to R22. An outer circumference on the camera module 51 side of the time lapse unit 50 is provided with a seal part of a silicone rubber, and, when the time lapse unit 50 is closed, the culture chambers R11 to R22 are kept in an airtight state at a predetermined atmospheric pressure or less by this seal part.

The embryo culture device 10 receives a supply of a carbon dioxide gas (CO₂) and a nitrogen gas (N₂) from an external gas cylinder. Gas ports 31 and 32 to which these gases are supplied are provided on a back surface of the embryo culture device 10. Furthermore, filter ports 35 and 36 to which a filter 34 is attached are also provided on the back surface of the embryo culture device 10. This filter 34 removes a foreign material such as dust in outdoor air taken in by an unillustrated built-in pump. The embryo culture device 10 mixes the carbon dioxide gas CO₂ and the nitrogen gas N₂ input from the gas ports 31 and 32, and air input through the filter 34 at a predetermined rate to generate a gaseous mixture. The rate of each gas in the gaseous mixture is measured by an unillustrated sensor, and is kept at the same rate at all times.

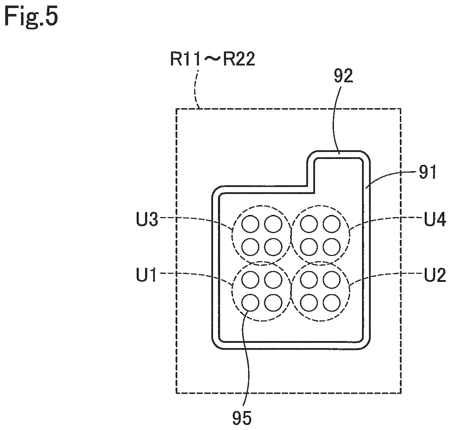

FIG. 3 is a plan view illustrating one of the culture chambers R11 to R22. As illustrated in FIG. 3, the culture chambers R11 to R22 are each provided with a dented tray accommodation part 40 in which a tray (also referred to as a dish) accommodating a treatment egg is placed. FIG. 5 illustrates a shape of a tray 91. The tray accommodation part 40 has the shape that matches the shape of the tray 91. According to the present embodiment, the tray accommodation part 40 has the shape (expanded part 42) whose one portion of the recess of a rectangular shape is expanded outward, and the tray 91 also has the shape (protrusion part 92) whose one portion protrudes outward likewise. Hence, the tray accommodation part 40 has the shape that is not symmetrical with respect to a center line on any one of front, back, left, and right sides. Hence, when the tray 91 is placed in the tray accommodation part 40, no mistake occurs regarding a direction in which the tray 91 is placed. Mistake of placement may be prevented by another method such as electrical detection, and the shapes of the tray accommodation part 40 and the tray 91 may be symmetrical with respect to the left and right sides and/or the front and rear sides. Furthermore, the tray 91 may be provided with a marker, and, arrangement (such as an orientation) of the tray 91 may be specified or the treatment egg may be directly specified from a captured image irrespectively of a placement direction.

A supply port 43 and a discharge port 44 may be provided in each of the bottom surfaces of the culture chambers R11 to R22. The supply port 43 is an opening for supplying a gaseous mixture to the culture chambers R11 to R22. Furthermore, the discharge port 44 is an opening for refluxing the gaseous mixture. Although, as described above, when the time lapse unit 50 is closed, the culture chambers R11 to R22 are placed in a substantially airtight state, and therefore a gas environment inside is kept constant, the gaseous mixture is supplied from the supply port 43 and discharged from the discharge port 44 to make temperature distributions in the culture chambers R11 to R22 uniform. Note that part of the gaseous mixture slightly leaks from a seal part sealed with the time lapse unit 50. By so doing, outdoor air is prevented from entering from the seal part sealed with the time lapse unit 50.

Each of the culture chambers R11 to R22 is provided with panel heaters H11 to H22 in a sidewall and a bottom surface of each of the culture chambers R11 to R22. The heaters H11 to H22 may be provided only in the sidewall or only in the bottom surface. Alternatively, independent heaters may be provided to the culture chambers R11 to R22. Furthermore, independent heaters may be provided on a lid side, too. The panel heaters H11 to H22 keep the interiors of the culture chambers R11 to R22 at constant temperatures. Note that, although description is omitted, the culture chambers R11 to R22 are each provided with an unillustrated temperature sensor and gas concentration sensor, and, moreover, a humidity sensor and the like, so that it is possible to detect temperatures, gas concentration levels, and humidity levels in the culture chambers R11 to R22. By feeding back signals from these sensors, constant temperatures and constant humidity levels are eventually kept in the culture chambers R11 to R22, and gas concentration levels are also kept constant. Note that, by keeping the temperature, the humidity level, the gas concentration level, and the like of the gaseous mixture supplied from the supply port 43 constant instead of detecting the temperatures and the like in the culture chambers R11 to R22, an environment in the culture chambers R11 to R22 may be kept constant.

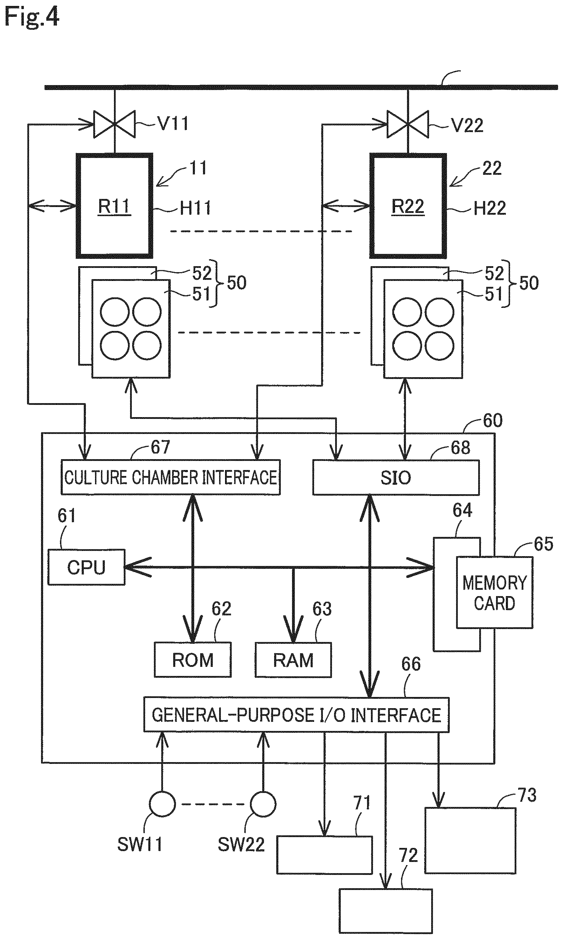

Next, the time lapse unit 50 will be described. FIG. 4 is an explanatory view illustrating an electrical configuration of the embryo culture device 10 including the time lapse units 50. As illustrated in FIG. 4, the embryo culture device 10 is provided with a control unit 60. The control unit 60 includes a known CPU 61, ROM 62, and RAM 63, and, in addition, a memory interface 64 that exchanges data with a memory card 65, a general-purpose I/O interface 66 that exchanges signals with external equipment, a culture chamber interface 67 that exchanges signals for controlling an environment in the culture chambers R11 to R22, an SIO 68 that performs serial communication with the time lapse units 50, and the like. The memory card 65 stores information related to an environment such as the temperature in each of the culture chambers R11 to R22.

The general-purpose I/O interface 66 is connected to the switches SW11 to SW22 that are provided to the embryo culture device 10, a driving device 71 that opens and closes the time lapse unit 50, a warning device 72 that issues a warning sound, a gaseous mixture adjustment device 73 that adjusts a gaseous mixture, and the like. The gaseous mixture adjustment device 73 includes a pressure adjustment valve that adjusts a pressure of a carbon dioxide gas, a nitrogen gas, or the like supplied to the gas ports 31 and 32, a pump that takes in an atmosphere through the filter 34, a pump that feeds the gaseous mixture to the culture chambers R11 to R22, and the like. The culture chamber interface 67 is connected with the panel heaters H11 to H22 that are provided to each of the culture chambers R11 to R22, control valves V11 to V22 that control supply amounts of gaseous mixtures, and the like. The control valves V11 to V22 are provided to pipes that reach the culture chambers R11 to R22 from a gaseous mixture supply pipe 84 that supplies the gaseous mixture.

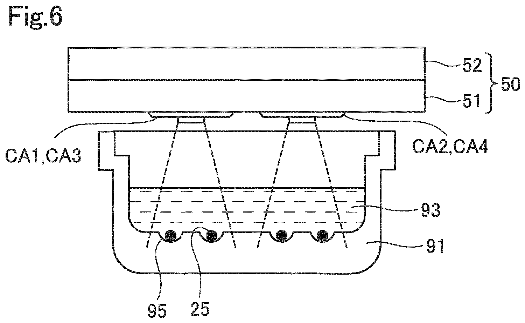

It has been already described that the camera module 51 of the time lapse unit 50 is provided with the four camera units CA1 to CA4. As illustrated in FIGS. 5 and 6, these camera units CA1 to CA4 each image wells 95 that are recess parts provided to the tray 91. Every four wells 95 are disposed collectively as a set at four portions in the tray 91 as illustrated. The camera units CA1 to CA4 are provided in association with respective sets. Areas U1 to U4 illustrated in FIG. 5 indicate respective imaging areas of the camera units CA1 to CA4. That is, each of the camera units CA1 to CA4 can image an area including the four wells 95 at once. Light guides that guide light from LEDs are provided around lenses of the camera units CA1 to CA4. The camera units CA1 to CA4 irradiate the trays 91 with light from the light guides at a time of imaging.

Each of the camera units CA1 to CA4 may image the more multiple wells 95 at once. For example, the camera unit CA1 may be provided at the center of the 16 wells 95, and the one camera unit CA1 may image the four areas U1 to U4 at once. Alternatively, only the camera unit CA1 and the camera unit CA4 may be provided, each camera unit CA1 may image the areas U1 and U2, and the camera unit CA4 may image the areas U3 and U4. Furthermore, the four camera units CA1 to CA4 may image the respectively adjacent areas U1 to U4 in an overlapping manner. By so doing, it is possible to acquire images of the wells 95 of all of the areas U1 to U4 by using a video image of the other camera unit even when one camera unit goes out of order and cannot capture an image as described below.

FIG. 6 schematically illustrates how imaging is performed. The well 95 is provided at a bottom part of the tray 91, and the one treatment egg 25 is basically put in the one well 95, and is coated with a mineral oil 93. Each of the camera units CA1 to CA4 images the areas U1 to U4 including the four wells 95 from above the tray 91. The captured images include images of the treatment eggs 25. The camera units CA1 to CA4 can adjust focus positions at several µm in accuracy at a time of imaging. How the camera units CA1 to CA4 image the treatment eggs 25 will be described later.

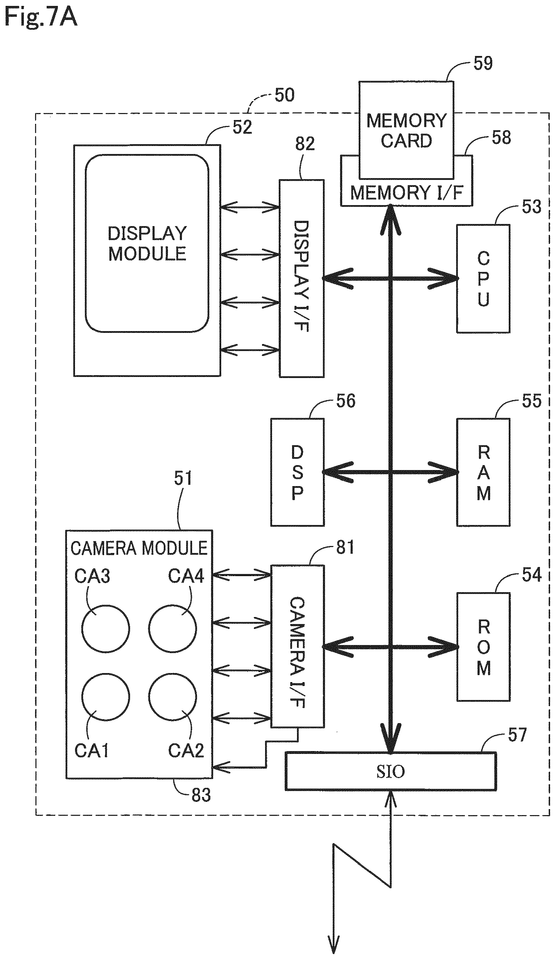

Next, an internal configuration of the time lapse unit 50 will be described. FIG. 7A is an explanatory view illustrating the internal configuration of the time lapse unit 50. As illustrated in FIG. 7A, the time lapse unit 50 includes the above-described camera module 51 and display module 52 and, in addition, a known CPU 53, ROM 54, RAM 55, a Digital Signal Processor (DSP) 56, an SIO 57, a memory interface 58, a camera interface 81, a display interface 82, and the like. The DSP 56 is a processor that can process a digital signal at a high speed, and employs a configuration that can process weighted operation processing suitable to machine learning over multiple layers.

A memory card 59 is attachable to the memory interface 58. This memory card 59 records images captured by the camera module 51 as described later. The display interface 82 is connected with the display module 52, outputs an image signal to be displayed on the display module 52, and receives an input of a signal from a touch panel provided to the display module 52. The camera interface 81 is connected with the camera module 51, performs processing of exchanging signals for controlling the focus positions of the respective camera units CA1 to CA4 of the camera module 51 and receiving signals of images captured by the respective camera units CA1 to CA4, and further outputs signals to the LEDs turned on at a time of imaging. Note that the "interface" such as the memory interface 58 is abbreviated as the "I/F" in the drawings.

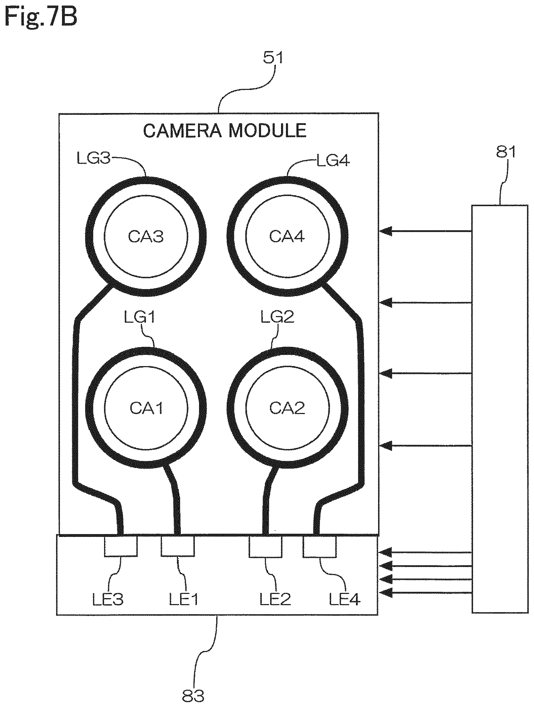

A light of the camera module 51 will be described with reference to FIG. 7B. As illustrated in FIG. 7B, an LED module 83 is attached to the camera module 51. The LED module 83 is provided with light emitting elements LE1 to LE4 that use illumination LEDs in association with the respective camera units CA1 to CA4. The light emitting elements LE1 to LE4 are each turned on and turned off according to a signal from the camera interface 81. The light emitting elements LE1 to LE4 are turned off by default. Note that the light emitting elements LE1 to LE4 may be light emitting elements such as organic ELs other than Light Emitting Diodes (LEDs). Furthermore, xenon lamps that are turned on at all times may be provided outside the time lapse unit 50, light of the xenon lamps may be brought into the time lapse unit 50 through optical fibers or the like, and guiding of the light to the camera module 51 may be turned on and off by a light switch.

Each of the light emitting elements LE1 to LE4 includes a light emission surface that faces toward the camera module 51, and this light emission surface is in contact with an end surface of each of light guide paths LG1 to LG4 formed in the camera module 51. The light guide paths LG1 to LG4 are so-called light guides, each include a core and a clad similar to the optical fiber, and guides light incident from the end surface to an annular terminal without leaking the light outside. The light of each of the light emitting elements LE1 to LE4 guided through each of the light guide paths LG1 to LG4 is emitted from the annular terminal to an imaging direction of each of the camera units CA1 to CA4, that is, a direction of the wells 95 in FIG. 6. A wavelength that does not influence the treatment egg 25 is selected for light emission from each of the light emitting elements LE1 to LE4. Note that the frequency of the light to be radiated on the well 95 is suitably a frequency at which a pronucleus that appears when the treatment egg 25 is fertilized is easy to see, that is, the frequency whose degree of absorption and reflection of light is significantly different at the pronucleus or a site other than the pronucleus.

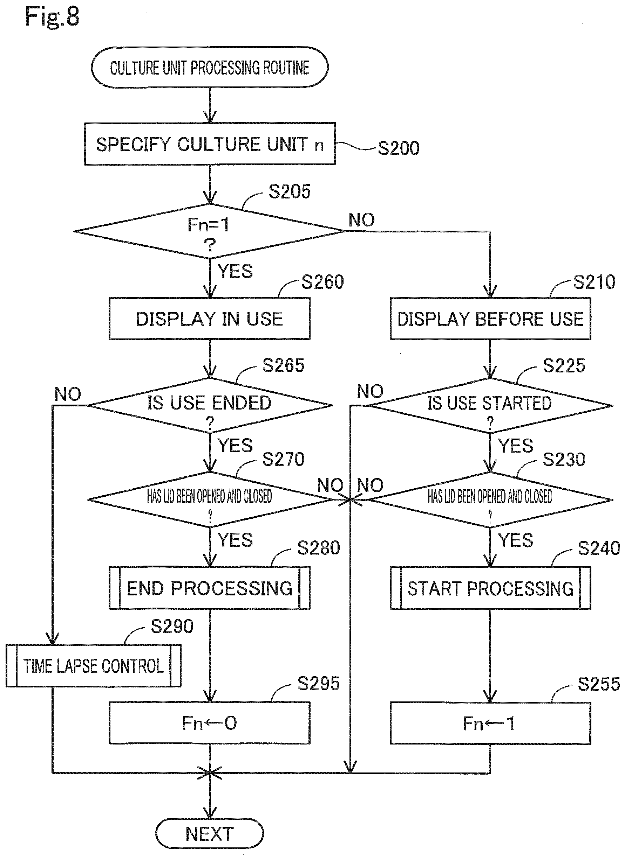

The above-described hardware configuration according to the first embodiment is the antecedent for the embryo culture device 10 to perform the following processing. According to the present embodiment, the control unit 60 of the embryo culture device 10 manages entire control, and the CPU 53 of the time lapse unit 50 manages imaging and display of the treatment egg 25. First, entire processing will be described with reference to FIG. 8. FIG. 8 is a flowchart illustrating a culture unit control routine. This processing is executed by the control unit 60.

The culture unit control routine illustrated in FIG. 8 is repeatedly executed at a predetermined interval when the embryo culture device 10 is powered on. When this processing routine is started, the culture unit for which processing is performed is determined first (step S200). In practice, a variable n for specifying the processing target culture units 11 to 22 is sequentially incremented between numerical values of 11 and 22 to specify the processing target culture unit. Note that the variable n is sequentially incremented, and, when the variable n reaches the value of 22, the variable n is set to the value of 11 at a time of next increment. That is, each of the culture units 11 to 22 becomes a processing target at a rate of one time in 12 times. When a number n of the processing target culture unit is determined in step S200, whether or not a flag Fn related to this culture unit n is a value of 1 is determined next (step S205). The flag Fn is reset to the value of 0 when the embryo culture device 10 is powered on.

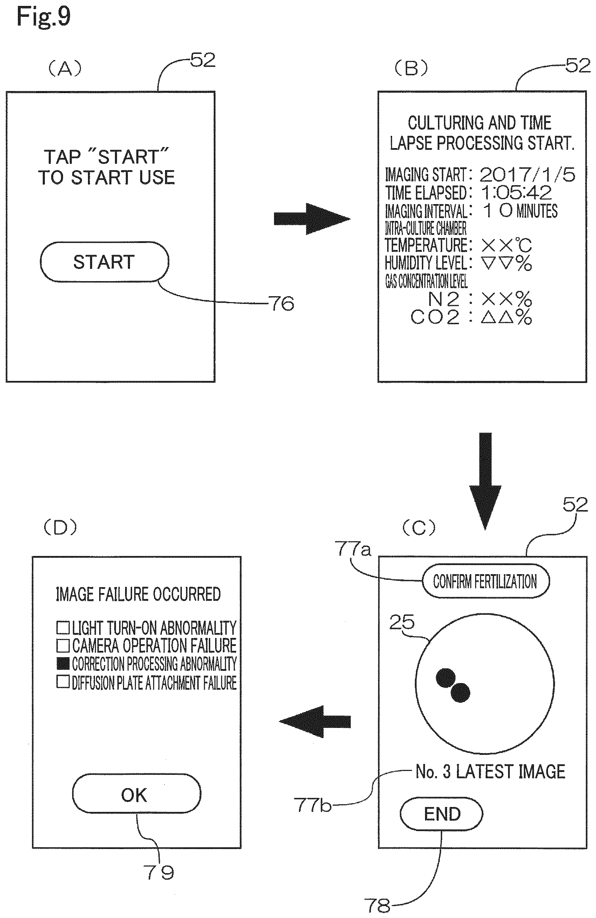

Hence, before each of the culture units 11 to 22 starts to be used, it is determined that the value of the flag Fn is not the value of 1, and pre-use display is performed next (step S210). The pre-use display is processing of transmitting a signal to the time lapse unit 50 of the processing target culture unit n via the SIO 68, and displaying that this culture unit n is before use. (A) in FIG. 9 illustrates an example of display. Hence, all of the display modules 52 of the time lapse units 50 of the pre-use culture units display explanation "tap 'start' to start use", and touch screens display "start" buttons 76.

Next, whether or not the "start" button 76 of the screen has been tapped is determined (step S225), and, in a case where the "start" button 76 has been tapped, whether or not the lid has been opened or closed is determined next (step S230). The lid, that is, the time lapse unit 50 is opened and closed when a switch SWn associated with each culture unit n to be used is operated. The switch SWn is operated to open the time lapse unit 50 of the culture unit n to be used, and dispose in a culture chamber Rn the tray 91 accommodating the treatment eggs 25 in the wells 95, and the switch SWn is operated again to close the time lapse unit 50. Thus, it is determined that the lid has been opened and closed.

In the case where the lid has been opened and closed, it is determined that the tray 91 has been disposed in the culture chamber Rn, start processing (step S240) is executed, then the flag Fn is set to the value of 1 (step S255), and this processing routine moves to "NEXT", and is temporarily ended. In this regard, the start processing (step S240) means processing of placing the temperature, the gas concentration, and the like in the culture chamber Rn in a desired state, and then instructing the time lapse unit 50 to start time lapse processing. Furthermore, that the flag Fn is the value of 1 indicates that the culture chamber Rn is in use. When the start processing is executed, the display module 52 is caused to display information on an interior of the culture chamber. Field (B) in FIG. 9 illustrates an example of this display. In this example, "culturing and time lapse processing start" is displayed, and information of the current culture chamber Rn is displayed below this message. More specifically, a start time and a time elapsed for imaging, information related to imaging such as an imaging interval, environment in the culture chamber, i.e., information of the temperature and the humidity, and, moreover, information of the gas concentration and the like are displayed. The temperature and the gas concentration are detected by the unillustrated sensor. In addition to these pieces of information, information for specifying a patient of a treatment egg such as a chart number or the like may be displayed.

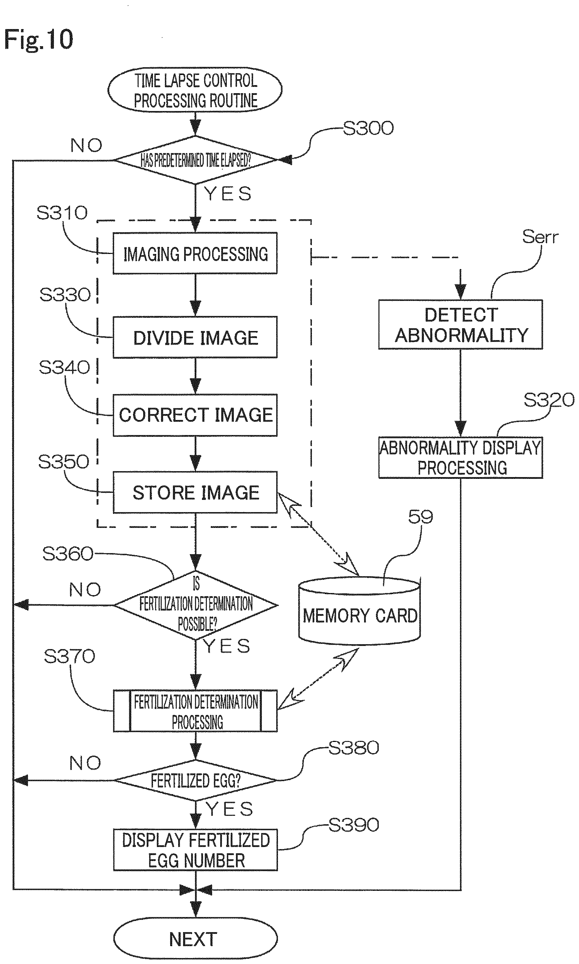

Culturing is started in this way, this processing routine is next activated when the flag Fn is set to the value of 1, the determination in step S205 is "YES" at the timing when the culture chamber Rn is specified in step S200, and processing moves to step S260. In step S260, the time lapse unit 50 is instructed to continue the time lapse processing. The time lapse processing executed by the time lapse unit 50 will be described later with reference to FIG. 10, yet, to put it simply, is processing of collectively imaging the plurality of respective wells 95 of the tray 91 in the culture chamber, dividing and storing images per well 95, individually determining a probability of fertilization of each treatment egg 25 in the well 95, and displaying a necessary image on the display module 52.

After the time lapse unit 50 is instructed to continue this time lapse processing, whether or not an instruction to end use has been accepted is determined (step S265). The instruction to end use can be obtained by an embryologist by operating the touch panel of the display module 52. When there is no instruction to end use, this processing routine moves to "NEXT" as is, and is temporarily ended. When there is the instruction to end use, whether or not the lid has been opened and closed is determined (step S270), and, after it is confirmed that the lid has been opened and closed, end processing (step S280) is executed. The end processing is processing of ending, for example, temperature control in the culture chamber Rn. Then, the flag Fn is set to the value of 0 (step S295), and this processing routine is temporarily ended.

Next, the time lapse processing will be described with reference to FIG. 10. FIG. 10 is a flowchart illustrating the time lapse control processing routine. This processing is repeatedly executed at the predetermined interval by the time lapse unit 50 that has started the start processing in step S240 in FIG. 8 until the end processing is performed in step S280.

When the time lapse control processing illustrated in FIG. 10 is started, whether or not a predetermined time has passed since an image of a treatment egg is previously captured is determined first (step S300). The predetermined time described herein corresponds to the imaging interval illustrated in field (B) in FIG. 9. The predetermined time may be a much shorter time or longer time instead of 10 minutes illustrated in field (B) in FIG. 9. In a case where the predetermined time has passed since previous imaging (step S300: "YES"), imaging processing is performed (step S310). The plurality of wells 95 are collectively imaged, that is, the respective camera units CA1 to CA4 collectively image the four wells 95 present in the areas U1 to U4. The captured images are stored together with data of an imaging time in the memory card 59.

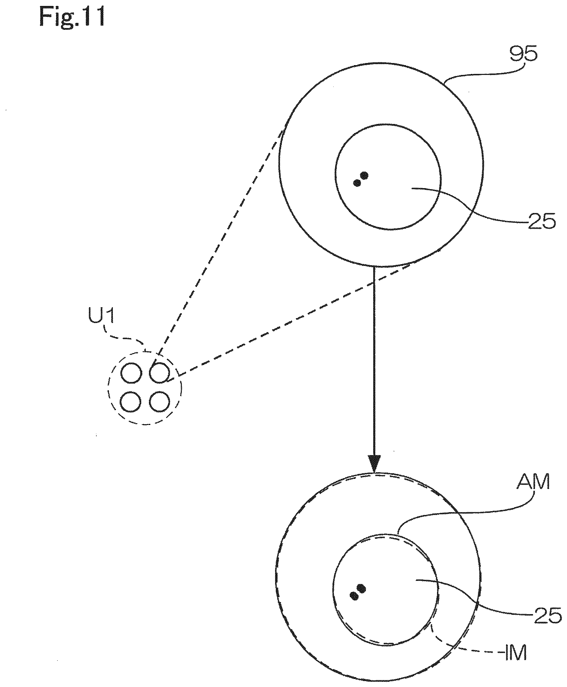

The imaging processing (step S310) is performed by simultaneously or sequentially driving the four camera units CA1 to CA4 provided in the camera module 51 of each time lapse unit 50. As illustrated in FIG. 5, by causing the respective camera units CA1 to CA4 to operate, states of the treatment eggs 25 accommodated in the 16 wells 95 of the tray 91 are imaged. FIG. 11 illustrates how the states of the treatment eggs 25 are imaged. The camera units CA1 to CA4 image the areas U1 to U4, respectively, and the areas U1 to U4 include the four wells 95.

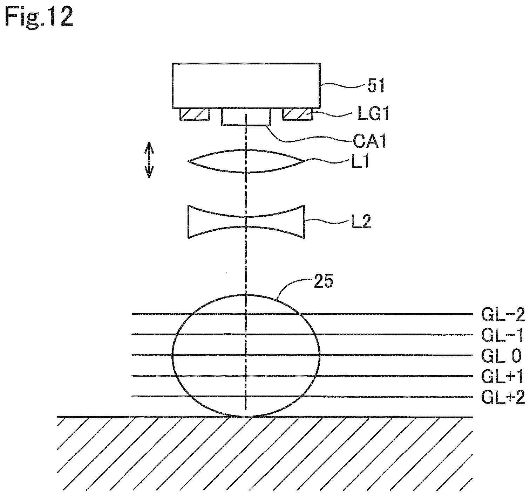

Each of the camera units CA1 to CA4 images the tray 91 from above. In this case, the camera units CA1 to CA4 image five images around the one well 95 while moving the focus positions. FIG. 12 illustrates how imaging is performed. Although FIG. 12 schematically illustrates lenses L1 and L2 outside the camera unit CA1, the lens L1 and L2 are actually built in the camera unit CA1. By moving at least one of the lenses L1 and L2 forward and backward along the optical axis, the focus position of the camera unit CA1 may change to a position GL+1 that is a on side distant from the camera unit CA1, a more distant position GL+2, a position GL-1 that is on a side closer to the camera unit CA1, and a closer position GL-2 with respect to a center position GL0. The size of the treatment egg 25 is approximately 100 to 150 µm in a case of an egg of a person, and therefore a difference between the respective positions is set to approximately 20 to 25 µm. In this case, a lens configuration of the camera units CA1 to CA4 has a shallow focal depth, and an imaging range at each focus position is adjusted to 25 to 30 µm. Each one of these camera units CA1 to CA4 captures five images. A plurality of images of different focus positions are captured because at which position of the treatment egg 25 that is a substantially spherical body a location of a pronucleus or the like in the treatment egg 25 that serves as a clue for determining fertilization is not known. Note that a lens configuration having a deep focal depth may capture a single image without changing the focus position in this way.

After the imaging processing, processing of dividing the captured image per well 95 is performed (step S330). Here, there are the respective four wells 95 in the areas U1 to U4 imaged by the respective camera units CA1 to CA4, and therefore the captured image is divided into four. Next, processing of correcting the divided images is performed (step S340). Correction is performed to remove distortion and dirt or debris from the images. The plurality of wells 95 are imaged at once, and therefore all of the wells 95 cannot be located right below the optical axes of the camera units CA1 to CA4. Hence, the image obtained by imaging each well 95 is distorted. In a case of a color image, there is also a case where aberration causes blur or color-tint.

Then, image correction (step S340) corrects such distortion, dirt or debris, and the like. FIG. 11 is an explanatory view illustrating an example of correction. When each one of the wells 95 in the area U1 is divided and taken out, the well 95 is not right below the optical axis, and therefore the outline of each well 95 formed in a true circle is distorted and does not become the true circle. Naturally, similar distortion occurs in the captured images of the treatment eggs 25, too. When the distorted image is corrected as indicated by broken lines in FIG. 11, the outline of each well 95 becomes the substantially true circle. The shape of the treatment egg 25 is also corrected likewise to the original shape. Such correction is performed not only to correct distortion of the shape, but also to remove color-tint due to chromatic aberration, a ghost caused by dirt or debris on a lens, and the like.

After image correction, if correction can be performed, processing of storing the image in the memory card 59 is performed (step S350). In this case, the image is stored per divided image. A tag for determining which one of the camera units CA1 to CA4 has captured each divided image may be attached as a file name or the like to each divided image to link. Furthermore, when each image is stored, only a corrected image may be stored, or the corrected image may be stored together with a pre-correction image or separately from a pre-correction image. Storage is not limited to the memory card 59, and each image may be stored on, for example, a server on a cloud. A storage destination is not limited to a memory card, and may be other storage media such as hard disks and SSDs.

During the above-described series of processing, that is, processing from imaging of the areas U1 to U4 by the camera units CA1 to CA4 to dividing, correcting, and then storing of each image, the CPU 53 monitors whether or not some abnormality has occurred (step Serr). Whether or not the abnormality has occurred is occasionally determined by interruption processing.

When it is detected that some abnormality has occurred from imaging to storage (steps S310 to S350) (step Serr), abnormality display processing (step S320) of displaying contents of an abnormality on the display module 52 is performed. In this case, the display module 52 functions as a presentation unit that presents an abnormality. Field (D) in FIG. 9 illustrates an example of abnormality display. In the illustrated example, the contents of abnormality are exemplified as, for example,
Light turn-on abnormality
Camera operation failure
Correction processing abnormality, and
Image division or storage abnormality
When these abnormalities occur, corresponding portions are marked and displayed. When overlapping abnormalities occur, a plurality of items are marked. In the example illustrated in field (D) in FIG. 9, "correction processing abnormality" is marked.

"Light turn-on abnormality" among the abnormalities refers to a case where each of the light emitting elements LE1 to LE4 of the above-described LED module 83 is not turned on, a case where light does not arrive due to an abnormality of the light guide paths LG1 to LG4, and a case where the wells 95 are not normally illuminated. In this case, the captured image becomes dark, and average brightness of an image does not fall within a predetermined range, so that it is possible to determine the abnormality. The camera operation failure refers to, for example, a case where the camera units CA1 to CA4 do not operate, and a case where light reception modules of a CCD and a CMOS for imaging do not operate. When an optical focus adjustment device and a shutter are provided, operation failures of these devices are also included. As for the light reception modules, the camera operation failure can be determined when, for example, a readout clock is not inputting normally. Note that, in the present embodiment, the camera unit CA1 is provided with a diffusion plate that uniformizes illumination light that is emitted from the light emitting elements LE1 to LE4, passes through the light guide paths LG1 to LG4, and illuminates the wells 95, and for example, a case where illumination light is not uniform due to an attachment direction of this diffusion plate or an attachment mistake of top and back sides of this diffusion plate is included in the light turn-on abnormality.

The correction processing abnormality refers to a case where correction for removing distortion and dirt or debris from a captured image is not normally performed. For example, the distortion is removed by inputting a gap from the optical axis or the like as a parameter in advance, and correcting a shape using this parameter, and, in a case where the shape of the well 95 is significantly different from the true circle as a result of correction, it is determined that correction processing has not been normally performed. Furthermore, although the processing of removing dirt or debris is normally performed by removing approximately one or two isolated dots on a screen, in a case where the number of isolated dots is a predetermined number or more, it is determined that the correction processing has not been normally performed.

The division abnormality of the image division or storage abnormality refers to, for example, a case where, when an image obtained by imaging the plurality of wells 95 is divided, and division processing of the image is normally ended, or an imaging center of the camera unit CA1 or the like is deviated and each well 95 does not fit in each divided image as a result of division. The divided image storage abnormality refers to, for example, a case where, when a file of a divided image is stored in the memory card 59 or the like, the file cannot be normally stored due to an insufficient capacity, a failure, or the like of the memory card 59.

When there is no abnormality in particular after the above processing (steps S330 to S350) is ended, whether or not a timing at which determination of fertilization can be made has come is determined (step S360). Determination of fertilization means determining whether or not the treatment egg 25 subjected to fertilization treatment has been fertilized. Fertilization generally succeeds until approximately eight hours pass at the earliest, approximately 22 hours pass on average, or 36 hours pass at the latest after the fertilization treatment. The treatment egg 25 subjected to the fertilization treatment is quickly accommodated in the well 95 of the tray 91 after the treatment, and is placed in one of the culture units 11 to 22. Therefore, when approximately six hours pass after the time lapse unit 50 starts imaging, determination in step S360 is "YES", that is, determination of fertilization is possible. This time may be made much shorter, and determination of fertilization may be possible immediately after the trays 91 are accommodated in the culture chambers R11 to R22. In this case, the determination in step S360 is unnecessary.

When it is determined that determination of fertilization is possible, fertilization determination processing is performed next (step S370). This processing enables determination by reading the image of the treatment egg 25 divided per well 95 and stored in the memory card 59, and determining whether or not two pronuclei are clearly captured in the image. This determination may be performed based on a change over time, more particularly, a change in the number of sites that can be recognized as the pronuclei by reading multiple images stored in the memory card 59 so far. Furthermore, multiple images of fertilized treatment eggs and multiple images of treatment eggs that have not been fertilized may be prepared, and subjected to machine learning in advance to input the images read from the memory card 59 to a determination device that has already performed this machine learning, and thereby determine whether or not fertilization has succeeded. Such processing can be achieved in real time by using a high-speed processing function of the DSP 56 illustrated in FIG. 7A. Note that the captured image may be displayed on the display module 52 to let the embryologist determine whether or not fertilization has succeeded.

After the fertilization determination processing (step S370) is executed, whether or not the treatment egg 25 is a fertilized egg from which fertilization can be confirmed is determined (step S380). When the treatment egg can be determined as the fertilized egg, fertilized egg number display (step S390) is performed. Field (C) in FIG. 9 illustrates an example of this display. In this example, a message 77a indicating that fertilization has been confirmed, the image of the treatment egg 25 from which fertilization has been confirmed, number display 77b indicating what number of the well 95 this fertilized egg is placed, and an "end" button 78 for instructing to end screen display are displayed on the display module 52. Subsequently, this processing routine moves to "NEXT" and is ended.

Assuming that imaging is started and there is a fertilized egg, if there is no abnormality in particular until the image of the fertilized egg is displayed, display that changes from field (A) to field (C) via field (B) is performed in the example in FIG. 9. If some abnormality occurs from imaging to storage, field (D) is displayed. Note that an abnormality needs to be detected not for all of the above-described items, but for at least one of the above-described items. An abnormality other than the above-described items may be detected and broadcast. In this case, the abnormality may be broadcast by being displayed on a display unit, or may be broadcast by a voice using an unillustrated voice output device. Furthermore, there may be adopted a mode for giving notification with an email or a message to a mobile phone of a person in charge.

Although the imaging processing performed at the predetermined interval and the fertilization determination processing in the processing illustrated in FIG. 10 have been described with reference to one flowchart for ease of description, both of the processing may be performed by separate processing routines in an asynchronous manner.

A display time in a case where imaging and fertilization determination are performed as a series of processing will be studied. The 2×2×4 (16 in total) wells 95 are provided in the tray 91 placed in the one culture chamber Rn, and the 16 treatment eggs can be accommodated at maximum. The time lapse unit 50 can simultaneously image these 16 wells 95 using the four camera units CA1 to CA4. Here, a case will be studied where, while the treatment eggs accommodated in these 16 wells 95 are sequentially displayed on the display module 52, the above fertilization determination is made and a fertilization determination result is displayed. The example of display in a case where it is determined that a treatment egg has been fertilized has already been illustrated as field (C) in FIG. 9. However, in a case where it is not possible to determine that the treatment egg has been fertilized, the latest image at a time of this determination among images of the treatment eggs may be displayed or may not be displayed. Assuming that the imaging interval is 10 minutes, each well 95 can be displayed for approximately 50 seconds. Consequently, the embryologist can learn the number of the well 95 accommodating the treatment egg that could have been fertilized by looking at this display. Furthermore, it is also possible to determine the end of culturing.

Performing insemination treatment simultaneously is rare. Therefore, even when there are the 12 culture units 11 to 22 in the embryo culture device 10 according to the present embodiment, the fertilized eggs are not necessarily displayed simultaneously in the 12 culture units in the entire embryo culture device 10. Consequently, in a case where only the fertilized eggs that have been determined to be fertilized are displayed on the display module 52, even when there are the 12 culture units 11 to 22, the embryologist can confirm fertilization looking at an image of this fertilized egg to be sequentially displayed, take out the tray 91, and perform subsequent treatment.

The above-described embryo culture device according to the first embodiment includes the time lapse unit 50 that is provided to each of the 12 culture chambers R11 to R22, and can collectively image the treatment eggs 25 respectively accommodated in the plurality of respective wells 95 of the tray 91 at the predetermined interval, divide the image into an image per well 95, and correct the images. Consequently, it is possible to perform fertilization determination based on the corrected images, and more accurately make a determination of fertilization. Furthermore, the image divided per well 95 is stored, so that it is possible to read only the image of the well 95 of interest. Consequently, it is possible to reduce a probability that the treatment egg 25 of the another well 95 is used as a fertilization determination target or the like by mistake.

According to the above embodiment, in a case where an abnormality occurs during one of imaging of the wells 95 by the camera units CA1 and CA2 (FIG. 10 and step S310), subsequent division of an image (step S330), correction of the image (step S340), and storage of the image (step S350), this abnormality is detected, and displayed on the display module 52. Consequently, it is possible to immediately learn of the abnormality when some abnormality occurs during acquisition of the image of the well 95. Hence, it is possible to avoid that it is not possible to obtain the image of the treatment egg 25 in the well 95 over a long period of time and make a determination of fertilization, or it is difficult to make a determination of fertilization. A user who has learned of the abnormality can take measures of, for example, repairing the embryo culture device 10, replacing the camera unit, exchanging the time lapse unit 50 itself, or moving the tray 91 to the other culture chambers R11 to R22, and continue imaging, dividing, correcting, or storing the treatment egg 25.

According to the above embodiment, it is not necessary to further move the tray 91 or the imaging device (such as the camera) to image the treatment egg 25. Consequently, the embryo culture device 10 not only can be made smaller, but also does not include a movable part, so that it is possible to enhance reliability of the embryo culture device 10. Furthermore, neither vibration nor noise occurs. Furthermore, it is possible to shorten the interval to image the treatment egg 25. In a case where the system that moves and images the tray 91 takes, for example, m minutes for moving and imaging the tray, 12×m minutes are required to move and image all of the 12 trays 91. That is, the interval cannot be made shorter than this time of 12 × m minutes. On the other hand, in the embryo culture device 10 of the present embodiment, the imaging interval can be shortened as much as the imaging interval of the camera units CA1 to CA4. In addition, it is also possible to continuously perform imaging.

Furthermore, according to the present embodiment, the one time lapse unit 50 is prepared for the one tray 91 accommodated in one of the culture chambers R11 to R22, this time lapse unit 50 is provided with the display module 52, and the image of the treatment egg 25 in the tray 91 accommodated in one of the culture chambers R11 to R22 is displayed thereon. Hence, there is also an advantage that the image displayed on the display module 52, and the tray 91 in the state where the switch SWn is operated and the time lapse unit 50 is opened have a one-to-one relationship, and the relationship between the image and the treatment egg is very clear.

Furthermore, according to the present embodiment, the time lapse control processing is performed per time lapse unit 50, so that it is possible to individually set time lapse processing such as an interval for imaging, a format of an image to display, and the like per time lapse unit 50 of each of the culture units 11 to 22. In a case of, for example, a treatment egg whose development after insemination treatment is of interest in particular, it is also easy to take measures of shortening the imaging interval, or asking for the embryologist to make a determination when whether or not fertilization succeeds is determined.

Furthermore, the time lapse unit 50 according to the present embodiment each includes the memory card 59, and divides and records images captured at the predetermined interval per well 95. Consequently, an image showing that, for example, a treatment egg cultured in this culture chamber Rn is fertilized is not taken as an image of another treatment egg by mistake. If necessary, captured images can be, for example, managed, stored, and discarded per memory card, and easily handled.

### B. Other Embodiment

While the time control processing is executed by the time lapse unit 50 alone in the above-described embodiment, entire processing including the time lapse control processing, too, may be performed by the control unit 60. Furthermore, whether or not the treatment egg 25 has been fertilized is determined in the above embodiment. However, such determination may not be made or may be made, then the treatment egg 25 continues to be cultured, and how the treatment egg 25 is cultured may be imaged by the camera units CA1 to CA4 that are the imaging units. Alternatively, a device may be configured to entirely automate accommodation of the treatment eggs 25 in the wells 95 of the trays 91 to delivery of the fertilized eggs that have been fertilized to next treatment.

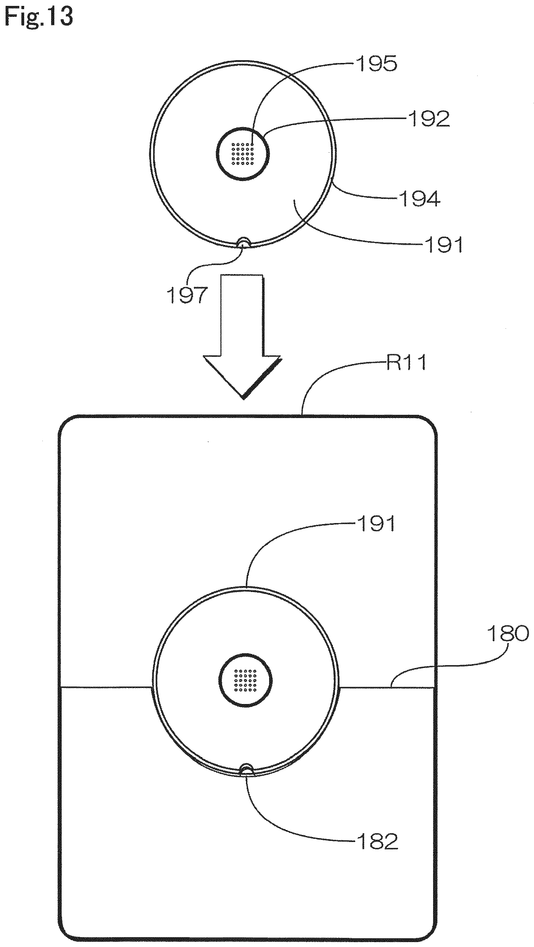

The four camera units CA1 to CA4 have been prepared per one time lapse unit 50 in the above embodiment. However, the number of camera units per time lapse unit 50 is random. The number of the wells 95 imaged by the camera units, that is, the number of treatment eggs to be imaged at once may be one, yet may be a random number equal to or more than two. Furthermore, an arrangement of the wells in the tray 91 is also random. For example, p×q wells may be aligned in an array pattern (one of p and q is a positive integer equal to or more than two). FIG. 13 is an explanatory view illustrating a dish 191 including 5×5, that is, 25 microwells 195 in total accommodating treatment eggs. While an upper part in FIG. 13 illustrates the dish 191, this dish 191 is accommodated at an accommodation portion of the culture chamber R11 in a state where the treatment eggs are accommodated in the microwells 195. The dish 191 has a cylindrical shape whose diameter is approximately 35 mm to make handling easy, and includes at the outer circumference an outer circumferential wall 194 whose height is approximately 10 mm. Furthermore, the dish 191 is provided with a circular partition wall 192 of approximately 2 mm to surround the microwell 195. An inside of this partition wall 192 is filled with a culture medium. The treatment egg is accommodated in the microwell 195 filled with the culture medium.

By so doing, at a time of handling of the dish 191, it is easy for the embryologist to carry the dish 191, and the culture medium only needs to fill small sections partitioned by the partition wall 192, so that the culture medium is not wastefully used. Furthermore, the 25 microwells 195 that accommodate the treatment eggs are accommodated in a several millimeter square, so that one camera unit can easily image the 25 microwells 195. When an imaging area is narrow, it is possible to increase resolution of an image per treatment egg, and enhance accuracy of determination of fertilization or the like.

The dish 191 illustrated in FIG. 13 includes a recess part 197 at one portion on an outer side of the outer circumferential wall 194. This recess part 197 is used to fit to a protrusion 12 provided to a holding frame 180 that holds the dish 191 and position the dish 191 at a predetermined position when the circular dish 191 is accommodated in the culture chamber R11. The holding frame 180 is formed in a shape that can accommodate an approximately half circumference of the dish 191, and the dish 191 is stably held at a predetermined position in the culture chamber R11. Note that the dish 191 is not limited to the circular shape, and may have other shapes such as a rectangular shape, an elliptical shape, and a trapezoidal shape. Furthermore, the partition wall 192 is not limited to the circular shape, and may have other shapes such as a square shape. Furthermore, the entire dish 191 may be filled with the culture medium without providing the partition wall 192.

In addition, the microwells 195 may be aligned in other alignment patterns such as an annular pattern. When the desired resolution can be obtained, any number of treatment eggs may be imaged at once. Contents and a method of correction and image division may be changed based on arrangement of the wells 95 and the microwells 195. Furthermore, a plurality of camera units the number of which is smaller than the number of trays may be provided, may move within the time lapse unit 50 and share imaging of the plurality of trays between the camera units.

The one tray 91 is disposed in the one culture chamber Rn in the above embodiment. However, two or more trays may be disposed in the one culture chamber Rn. In this case, the one time lapse unit 50 may be associated with the plurality of trays, and the one time lapse unit 50 may be associated with the one tray.

The time lapse unit 50 and the camera module 51 are also disposed as a lid at the upper part of the culture chamber Rn in the above embodiment. However, the lid of the culture chamber Rn may be provided separately from the camera module 51. Furthermore, the camera module 51 can be also disposed on a bottom surface or a side surface of the culture chamber Rn. In a case where the camera module 51 is disposed on the bottom surface, the bottom surface of the culture chamber Rn may be made of a material such as transparent glass, the camera unit CA1 or the like may be disposed below the bottom surface, and the treatment egg in the well 95 of the tray 91 may be imaged. Generally, the treatment egg sinks to the lowermost part in the well 95, and therefore it is easy to adjust the focus of the camera unit to the treatment egg by imaging the treatment egg from below the floor surface. In this case, only the display module 52 may be disposed as the lid at the upper part of the culture chamber Rn, or the display module 52 may be provided at another place.

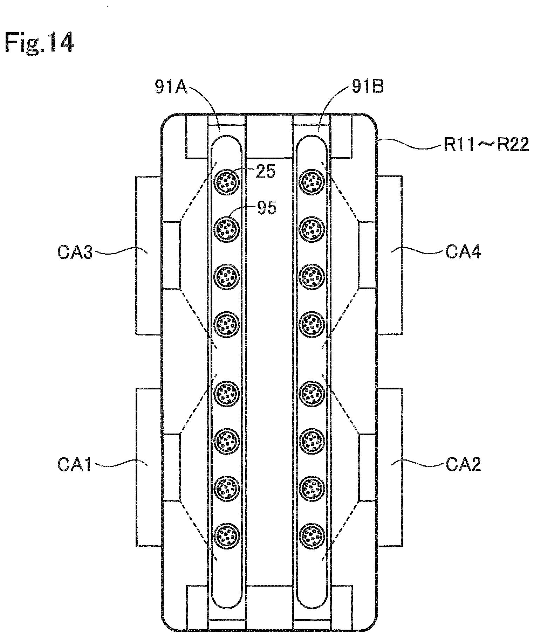

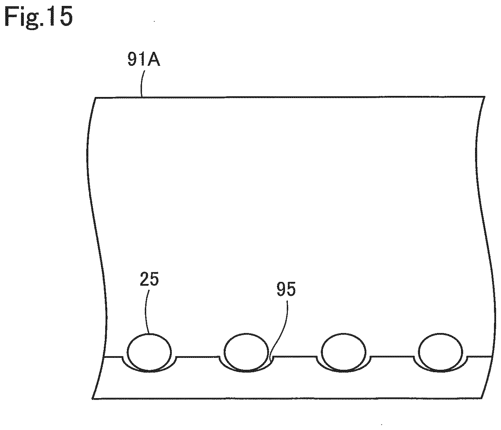

The camera module 51 may be provided on the side surface of the culture chamber Rn. In this case, the wells of the tray are linearly arranged. FIG. 14 illustrates a relationship between trays 91A and 91B and the culture chamber Rn in a case where the camera module 51 is provided on the side surface. As illustrated in FIG. 14, the eight wells 95 are linearly disposed in each of the trays 91A and 91B. Every four treatment eggs 25 in the eight wells 95 disposed in a row in the tray 91A are imaged by the two camera units CA1 and CA23 of a camera module 51A provided on the left side surface of the culture chamber Rn. The rest of the two camera units CA2 and CA4 of the camera module 51A are provided on the right side surface of the culture chamber Rn, and every four of the eight treatment eggs of the tray 91B are imaged. FIG. 15 schematically illustrates a state where this tray 91A is seen from the side of the camera unit CA1. The wells 95 are provided on the bottom surface of the tray 91A, and the treatment eggs 25 are accommodated therein. Consequently, a positional relationship between the camera units CA1 to CA4 and the treatment eggs 25 becomes stable, so that it is possible to easily capture images of the treatment eggs 25.

In this example, it is possible to reduce the widths of the trays 91A and 91B disposed in the one culture chamber Rn, so that it is possible to dispose the multiple culture chambers Rn and trays 91A and 91B in the one embryo culture device 10. Although, when the widths are narrowed, the trays 91A and 91B and the camera units are disposed close to each other, it is possible to clearly capture an image by using wide angle lenses even when the trays 91A and 91B are placed close to each other. When the wide angle lenses are used, distortion of an image becomes greater toward the periphery, and therefore it is necessary to greatly correct the image closer to the periphery in proportion to the distortion. Note that the camera module 51 may include camera units on front and rear side surfaces instead of the left and right side surfaces or in addition to the left and right side surfaces. The camera units may be provided on one of the left, right, front, and rear side surfaces. Alternatively, the camera units may be provided on the upper surface and the bottom surface to image treatment eggs from a plurality of directions.

When one treatment egg is imaged, a focus position is shifted, and a plurality of images are captured in the above embodiment. However, only one image of the one treatment egg may be captured at the predetermined interval without shifting the focus position in particular. Note that imaging may be performed using not only visible light, but also a range of near infrared, far infrared, ultraviolet light, or the like. Alternatively, a filter that allows only a predetermined wavelength range of visible light to transmit through may be provided, and imaging may be performed using only light that transmits through the filter.

When the wells 95 are illuminated, the wells 95 are illuminated from sides of the camera units CA1 to CA4 in the above embodiment. However, the embodiment is not limited to this configuration, and the wells 95 may be illuminated from the side or from below. In the embodiment, the light emitting elements LE1 to LE4 that are the light sources are provided in the LED module 83 that is a place apart from the camera units CA1 to CA4 to guide light to the vicinity of the camera units CA1 to CA4 through the light guide paths LG1 to LG4. However, the light emitting elements may be provided near the camera units CA1 to CA4. Alternatively, by contrast with this, the light sources may be provided at any place of the embryo culture device 10 instead of the time lapse unit 50, and light may be guided to the time lapse unit 50 through an optical fiber or the like.

The display module 52 is provided per time lapse unit 50 in the above embodiment. However, the display module 52 may be provided separately from the time lapse unit 50. Furthermore, in this case, the display module 52 may be integrated with a form such as a large liquid crystal panel. Alternatively, a display of another computer or the like may be used to display using wireless LAN or wired LAN. Furthermore, an application may be installed in a mobile device such as a mobile telephone used by the embryologist as a display. By so doing, even when the embryologist is not in a room in which the embryo culture device 10 is placed, the embryologist can check a situation of a treatment egg in real time. Alternatively, a patient who has provided a treatment egg and is undergoing, for example, fertility treatment can check a situation of the treatment egg. Furthermore, by so doing, two or more of a plurality of embryologists, or an embryologist and a patient can simultaneously check the situations of treatment eggs. Consequently, the embryologists, or the embryologist and the patient do not miss a timing of fertilization.

The imaging timing (the start time or the interval) can be set per time lapse unit 50 provided per culture chamber Rn in the above embodiment. However, imaging start may be limited to a predetermined timing such as on the hour. Furthermore, the interval or the like may be also common between the plurality of time lapse units 50. Furthermore, a button displayed on the display module 52 is tapped to, for example, instruct imaging start. However, the instruction may be made by voice recognition together with other instructions. A dedicated instruction board may be prepared to operate a switch, a hardware button, or the like arranged on the instruction board.

In the above embodiment, the time lapse unit 50 also functions as the lid of the culture chamber Rn, is attached to a rim of the culture chamber Rn by an unillustrated hinge, and rotates to open and close the culture chamber Rn as illustrated in FIG. 2 to make it possible to attach and detach the tray 91. The time lapse unit 50 may be attached to the culture chamber Rn by a method other than rotation and movement. For example, the time lapse unit 50 may be simply placed on the upper surface of the culture chamber Rn. Alternatively, the time lapse unit 50 may be translated upward and downward. The time lapse unit 50 can be also configured to slide leftward and rightward or forward and backward.

The time lapse unit 50 is designed and attached as part of the embryo culture device 10 from the beginning in the above embodiment. However, the time lapse unit 50 may be exchanged with a lid of a culture chamber of an embryo culture device that has already been sold and installed. By so doing, the embryo culture device that has already been installed can be easily remade as an embryo culture device of a time lapse type.

The various embodiments of the present disclosure have been described above. However, the present disclosure is not limited to these embodiments, and can be naturally carried out as various embodiments without departing from the gist of the present disclosure. For example, the culture chamber Rn and the time lapse unit 50 may be paired as a minimum unit, these minimum units may be couplable in a left/right direction and/or a forward/backward direction, and a random number of culture chambers and a random number of time lapse units may be combined to form an embryo culture device. In the embryo culture device according to the present disclosure, the imaging unit is provided in association with each one of the trays held in the culture unit, so that, by coupling the imaging units and the trays in this way, it is easy to additionally install the culture units.

### C. Other Embodiment:

(1) Various embodiments of the present disclosure will be described below. A first aspect is an aspect of an embryo culture device that keeps in a culture environment, a treatment egg having been subjected to insemination treatment. This embryo culture device includes: a culture unit that accommodates a plurality of trays each including a plurality of recess parts that can each accommodate a treatment egg, and keeps the plurality of trays in the culture environment; a plurality of imaging units that are each provided in association with each one of the trays held in the culture unit, and continuously or intermittently image at least two or more of the plurality of recess parts among locations of the trays at once; a storage unit that divides and stores an image captured by each of the imaging units per area including the plurality of recess parts; a correction unit that corrects the stored image per recess part; and a determination unit that determines using the corrected image whether or not the treatment egg is fertilized. This embryo culture device can image the plurality of recess parts that can accommodate the treatment eggs at once using the imaging unit provided per tray, subsequently divide the image per recess part, and correct each divided image. Consequently, it is possible to correct the image of the treatment egg accommodated in each recess part while imaging the plurality of recess parts at once, so that it is possible to more accurately perform fertilization determination.
(2) According to this configuration, the correction may be a correction of matching positions of the recess parts with respect to the imaging units. By so doing, it is possible to correct distortion aberration caused in the images of the recess parts due to the positional relationship with the imaging units.
(3) According to this configuration, the correction may be a correction of matching an imaging condition of each of the trays provided with the imaging units. By so doing, it is possible to perform a correction that matches an imaging condition that differs per tray such as a difference in brightness of a light.
(4) According to this configuration, the imaging units may image all of the plurality of recess parts provided to the trays at once. By so doing, imaging only needs to be performed once, and it is possible to reduce the number of imaging units such as camera units required for imaging. Furthermore, it is possible to reduce the time required for imaging. This measure is possible because the correction unit corrects the image of each recess part.
(5) This configuration may further include a presentation unit that, when an abnormality occurs during the imaging, presents the contents of the abnormality caused. By so doing, it is possible to reduce a probability that occurrence of the abnormality during imaging is not noticed, and a mistake occurs in determination of fertilization. When an abnormality occurs during imaging, a cause of the abnormality may be found out to repair the abnormality, or the tray in the culture unit may be moved to the culture unit that is not in use to continue culturing and imaging.
(6) According to this configuration, the contents of the abnormality may be at least one of
   <1> that the imaging units cannot perform imaging,
   <2> that correction cannot be normally performed per imaging position,
   <3> that lights for imaging provided to the imaging units are not normally turned on, and
   <4> that the image cannot be normally divided or stored. By so doing, it is possible to support various abnormalities, and learn contents of an abnormality and handle the abnormality.
(7) According to this configuration, the culture unit may include a plurality of culture chambers partitioned per tray. By so doing, the one tray is associated with the one culture chamber, and it is possible to easily arrange the environment of the treatment egg in the tray.
(8) According to this configuration, the imaging units may be each provided on at least one surface of an upper surface, a side surface, and a bottom surface of each of the culture chambers facing the locations of the trays. By so doing, the imaging units only need to be provided on one of the surfaces, so that it is possible to increase the degree of freedom of design. Furthermore, it is possible to achieve appropriate imaging matching a state of a treatment egg.
(9) According to this configuration, the imaging units may each include an imaging focal depth change unit that captures a plurality of images of different focus positions during the imaging, and the storage unit may store the plurality of images of the different focus positions as a set of images. By so doing, it is possible to image details of states in a depth direction of the treatment eggs seen from the imaging units, so that it is possible to improve the accuracy of fertilization determination.
(10) This configuration may include a light guide that guides light from a light source, and irradiates with the light at least a location of the treatment egg on each of the plurality of trays. By so doing, it is possible to separate the light sources and the treatment eggs, so that the treatment eggs are hardly influenced by heat generated by the light sources. Furthermore, it is possible to freely design an arrangement of the light sources.
(11) This configuration may include broadcast units that are provided in association with the plurality of trays held in the culture unit, and, when the fertilization determination unit decides that the treatment egg in at least one of the recess parts has been fertilized, broadcast that the tray that accommodates the treatment egg determined to have been fertilized has been specified. By so doing, it is possible to easily specify that the treatment eggs have been fertilized, and specify the trays that accommodate the fertilized treatment eggs.
(12) A second aspect is an aspect of an imaging device that is attached to a culture unit of an embryo culture device that accommodates a plurality of trays each including a plurality of recess parts that can each accommodate a treatment egg having been subjected to insemination treatment, and keeps the plurality of trays in the culture environment. This imaging device includes: a plurality of imaging units that are provided in association with each one of the plurality of trays held in the culture unit, and continuously or intermittently image at least two or more of the plurality of recess parts among locations of the trays at once; a storage unit that divides and stores an image captured by each of the imaging units per area including the plurality of recess parts; a correction unit that corrects the stored image per recess part; and a display unit that displays the corrected image. This imaging device can image the plurality of recess parts that can accommodate the treatment eggs at once using the imaging unit provided per tray, then divide the image per recess part, and correct each divided image. Consequently, the image of the treatment egg accommodated in each recess part is corrected while imaging the plurality of recess parts at once, so that it is possible to more accurately learn a situation of the treatment egg.

The present disclosure is not limited to the above-described embodiments, and can be achieved by various configurations without departing from the gist of the present disclosure. For example, technical features in the embodiments corresponding to the technical features in each embodiment described in the Summary can be replaced or combined as appropriate to solve part or all of the above-described problems or achieve part or all of the above-described effects. Furthermore, unless these technical features are described as indispensable in this description, the technical features can be deleted as appropriate. For example, part of components achieved as hardware in the above embodiments can be achieved by software.

### Industrial Applicability

The embryo culture device and the imaging devices attached to the culture units of the embryo culture device according to the present disclosure can be used as devices that culture treatment eggs having been subjected to insemination treatment, perform fertilization determination, and as imaging devices that achieve said culturing and determination.

### Reference Signs List

CA1 ... CAMERA UNIT, H11 to H22 ... PANEL HEATER, L1, L2 ... LENS, LE1 to LE4 ... LIGHT EMITTING ELEMENT, LG1 to LG4 ... LIGHT GUIDE PATH, R11 to R22 ... CULTURE CHAMBER, SW11 to SW22 ... SWITCH, 9 ... CASE MAIN BODY, 10, 10A ... EMBRYO CULTURE DEVICE, 11 to 22 ... CULTURE UNIT, 25 ... TREATMENT EGG, 31 ... GAS PORT, 34 ... FILTER, 35, 36 ... FILTER PORT, 40 ... TRAY ACCOMMODATION PART, 42 ... EXPANDED PART, 43 ... SUPPLY PORT, 44 ... EXHAUST PORT, 50 ... TIME LAPSE UNIT, 51, 51A ... CAMERA MODULE, 52 ... DISPLAY MODULE, 53 ... CPU, 54 ... ROM, 55 ... RAM, 58 ... MEMORY INTERFACE, 59 ... MEMORY CARD, 60, 60A ... CONTROL UNIT, 61 ... CPU, 62 ... ROM, 63 ... RAM, 64 ... MEMORY INTERFACE, 65 ... MEMORY CARD, 66 ... GENERAL-PURPOSE I/O INTERFACE, 67 ... CULTURE CHAMBER INTERFACE, 71 ... DRIVING DEVICE, 72 ... WARNING DEVICE, 73 ... GASEOUS MIXTURE ADJUSTMENT DEVICE, 76 ... "START" BUTTON, 77 ... MESSAGE, 78 ... "END" BUTTON, 81 ... CAMERA INTERFACE, 82 ... DISPLAY INTERFACE, 83 ... LED MODULE, 84 ... GASEOUS MIXTURE SUPPLY PIPE, 91, 91A, 91B ... TRAY, 92 ... PROTRUSION PART, 93 ... MINERAL OIL, 95 ... WELL

## Claims

1. An embryo culture device that keeps in a culture environment a treatment egg having been subjected to insemination treatment, the embryo culture device comprising:
a culture unit that accommodates a plurality of trays each including a plurality of recess parts that can each accommodate a treatment egg, and keeps the plurality of trays in the culture environment;
a plurality of imaging units that are each provided in association with each one of the trays held in the culture unit, and continuously or intermittently image at least two or more of the plurality of recess parts among locations of the trays at once;
a storage unit that divides and stores an image captured by each of the imaging units per area including the plurality of recess parts;
a correction unit that corrects the stored image per recess part; and
a determination unit that determines using the corrected image whether or not the treatment egg is fertilized.

2. The embryo culture device according to claim 1, wherein the correction is a correction of matching positions of the recess parts with respect to the imaging units.

3. The embryo culture device according to claim 1 or 2, wherein the correction is a correction of matching an imaging condition of each of the trays provided with the imaging units.

4. The embryo culture device according to any one of claims 1 to 3, wherein the imaging units image all of the plurality of recess parts provided to the trays at once.

5. The embryo culture device according to any one of claims 1 to 4, further comprising a presentation unit that, when an abnormality occurs during the imaging, presents the contents of the abnormality caused.

6. The embryo culture device according to claim 5, wherein the contents of the abnormality is at least one of
<1> that the imaging units cannot perform imaging,
<2> that correction cannot be normally performed per imaging position,
<3> that lights for imaging provided to the imaging units are not normally turned on, and
<4> that the image cannot be normally divided or stored.

7. The embryo culture device according to any one of claims 1 to 6, wherein the culture unit includes a plurality of culture chambers partitioned per tray.

8. The embryo culture device according to claim 7, wherein the imaging units are each provided on at least one surface of an upper surface, a side surface, and a bottom surface of each of the culture chambers facing the locations of the trays.

9. The embryo culture device according to claim 8, wherein
the imaging units each include an imaging focal depth change unit that captures a plurality of images of different focus positions during the imaging, and
the storage unit stores the plurality of images of the different focus positions as a set of images.

10. The embryo culture device according to any one of claims 1 to 9, further comprising a light guide that guides light from a light source, and irradiates with the light at least a location of the treatment egg on each of the plurality of trays.

11. The embryo culture device according to any one of claims 1 to 10, further comprising broadcast units that are provided in association with the plurality of trays held in the culture unit, and, when the determination unit decides that the treatment egg in at least one of the recess parts has been fertilized, broadcast that the tray that accommodates the treatment egg determined to have been fertilized has been specified.

12. An imaging device that is attached to a culture unit of an embryo culture device that accommodates a plurality of trays each including a plurality of recess parts that can each accommodate a treatment egg having been subjected to insemination treatment, and keeps the plurality of trays in the culture environment, the imaging device comprising:
a plurality of imaging units that are provided in association with each one of the plurality of trays held in the culture unit, and continuously or intermittently image at least two or more of the plurality of recess parts among locations of the trays at once;
a storage unit that divides and stores an image captured by each of the imaging units per area including the plurality of recess parts;
a correction unit that corrects the stored image per recess part; and
a display unit that displays the corrected image.
